# EUROPÄISCHE PATENTSCHRIFT

(11) **EP 0 443 388 B1**
(45) Veröffentlichungstag und Bekanntmachung des Hinweises auf die Patenterteilung: **04.01.1995**
(21) Anmeldenummer: 91101813.3
(22) Anmeldetag: 09.02.1991
(51) Int. Cl.: A61F 5/04

(54) **Fixations- und Mobilisationsschiene**
Fixation and mobilization splint
Eclisse de fixation et de mobilisation

(30) Priorität: 23.02.1990 CH 591/90
(43) Veröffentlichungstag der Anmeldung: 28.08.1991
(73) Patentinhaber: Grob, Michael, Dr. med., CH-8640 Rapperswil (CH)
(72) Erfinder: Grob, Michael, Dr. med., CH-8640 Rapperswil (CH)
(74) Vertreter: Bosshard, Ernst

(56) Entgegenhaltungen:
- GB-A- 536 341
- US-A- 1 328 598
- US-A- 1 389 741
- US-A- 1 817 212
- US-A- 2 357 323
- US-A- 2 520 035
- US-A- 2 863 449
- US-A- 3 769 970
- US-A- 4 366 812
- US-A- 4 790 301

## Beschreibung

Die Erfindung betrifft eine Fixations- und Mobilisationsschiene gemäss Oberbegriff des Patentanspruchs 1.

Bekanntlich wird eine Fixationsschiene zum Ruhigstellen des Handgelenkes, des Daumens und der Finger verwendet. Ein solches Ruhigstellen empfiehlt sich nach Operationen, Fraktionen, Luxationen oder Ueberstreckungen. Eine Mobilisationsschiene wird zur Wiedererlangung der Bewegungen von Gliedmassen, insbesondere von Finger- und Daumengelenken verwendet. Eine kombinierte Fixations- und Mobilisationsschiene bringt den Vorteil, dass die Heilungs- und Gewöhnungsverläufe individuell für jedes Glied beschleunigt werden.

In dem CH-Patent Nr. 590.664 wird eine Hand- und Fingerorthese beschrieben, welche nur als Trainingsgerät die Bewegungsabläufe der Finger üben soll. Dies geschieht dadurch, dass die einzelnen Finger ohne Unterstützung nur an Fäden gehalten werden und somit keine eindeutige den Heilungsprozess fördernde Stellung einnehmen können. Ferner sind die Fäden an einer elastischen Feder aufgehängt, was die unkontrollierten Bewegungen der Finger noch unterstützt und den Heilungsprozess noch mehr verringert.

Das US-Patent Nr. 4.719.906 beschreibt eine kombinierte Fixations- und Mobilisationsschiene, welche für jeden Fingerknochen und Daumenknochen ein Stützglied besitzt, das mittels Klettenverschluss am Finger- bzw. Daumenknochen befestigt ist. Die zu einem Finger oder Daumen gehörenden Stützgliedteile sind untereinander durch Schrauben verbunden, so dass der gewünschte Winkel eines jeden Stützgliedteils einstellbar ist, nachdem die betreffenden Schrauben mit einem Schraubenzieher gelöst wurden. Der Winkel eines jeden solchen Teils wird anschliessend wieder fixiert. Hierbei ergibt sich der Nachteil, dass pro Gelenk zwei Schrauben zu fixieren sind. Dies Vorgehen hat sich in der Praxis als kompliziert erwiesen, da jeder Finger drei Gelenke mit zusammen sechs Schrauben besitzt, welche einzeln und relativ zueinander einzustellen sind. Ausserdem macht sich das Gewicht der aus Stahl bestehenden Fixations- und Mobilisationsschiene für den Träger unangenehm bemerkbar.

Die Erfindung hat die Aufgabe, die Nachteile der bekannten Schienen zu vermeiden und eine einfach konstruierte, wenig Gewicht aufweisende und eindeutige Winkelpositionen für Finger und Daumen enthaltende, kombinierte Fixations- und Mobilisationsschiene zu schaffen, wobei die Einzelbauteile praktisch ohne Werkzeug zusammengesetzt oder ausgetauscht werden können. Ferner erhöht die erfindungsgemässe Schiene den Tragekomfort für den Patienten, der öfters mehrere Wochen lang mit einer solchen Schiene die täglichen Verrichtungen ausüben muss.

Ausführungsbeispiele der Erfindung werden anhand der Zeichnungen näher erläutert.

Es zeigen:
- Figur 1: ein erstes Ausführungsbeispiel, dessen Konstruktionsteile zu einem Stück integriert sind;
- Figur 2: ein zweites Ausführungsbeispiel, dessen Konstruktionsteile zusammensetzbar sind;
- Figur 3: ein Teil des zweiten Ausführungsbeispiels;
- Figur 4: ein Stützglied für den Daumen über zwei Gelenke hinweg;
- Figur 5: ein Stützglied für einen Finger über zwei Gelenke hinweg;
- Figur 6: ein Stützglied für einen Finger bis zum ersten Glied;
- Figuren 7 - 13: verschiedene Verbindungsmittel zwischen Stützglieder und Handtellerstütze sowie Handtellerstütze und Daumenteil.

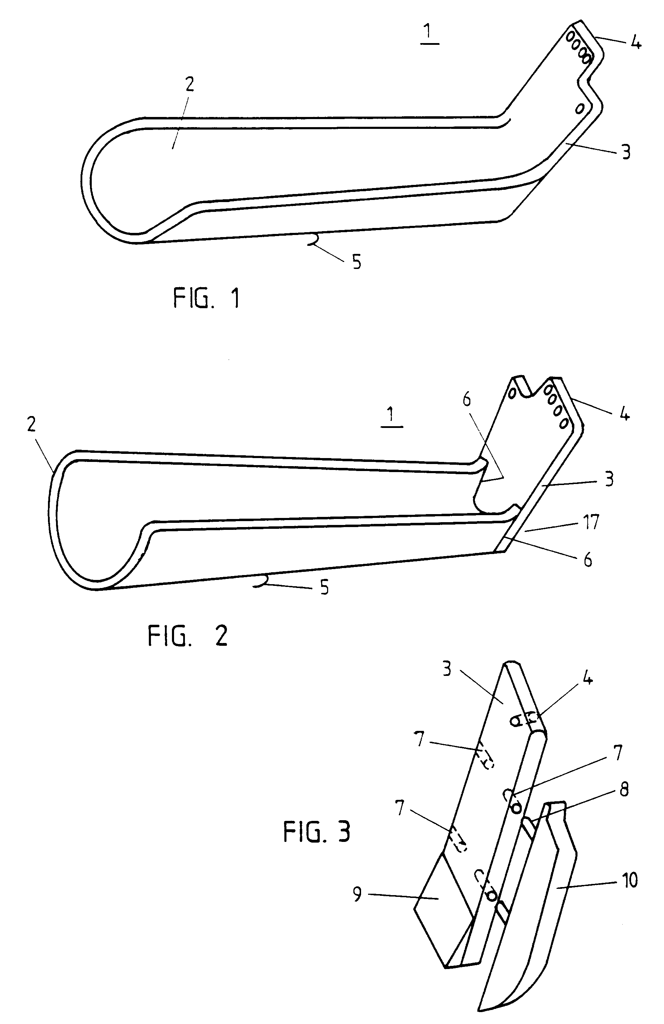

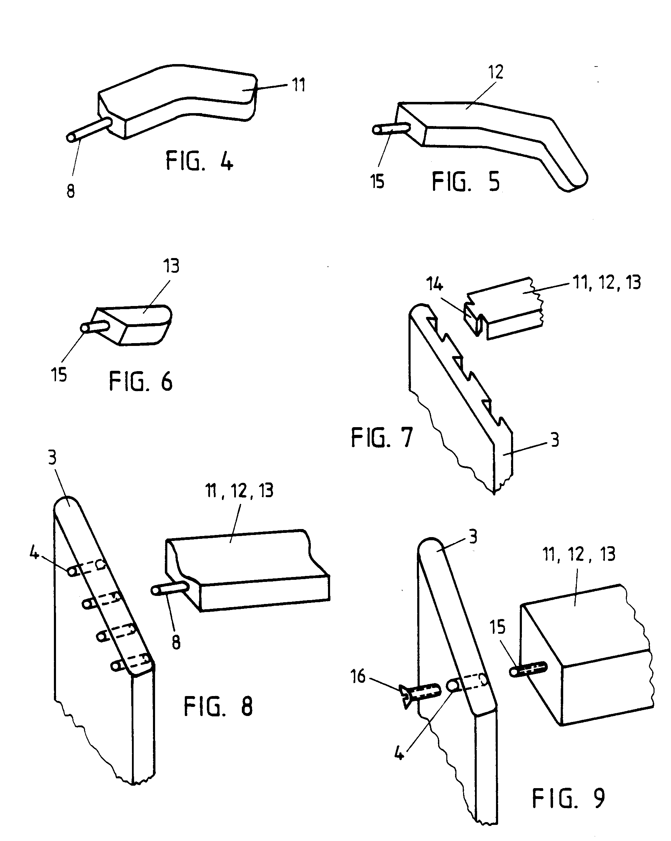

Gemäss Figur 1 besteht die Schiene 1 aus dem Teil 2, welcher am Unterarm des Patienten auf herkömmliche Art befestigt wird, und aus der Handtellerstütze 3, welche von der Handwurzel bis zu den ersten Fingergelenken bzw. zum ersten Daumengelenk reicht, und am oberen Rand Einrichtungen 4 zur Anbringung und Befestigung von in den Figuren 4, 5, 6 gezeigten Stützgliedern aufweist. Die Befestigungen sind in der gemäss den Figuren 7, 8, 9 gezeigten Art ausgebildet. In der Handtellerstütze 3 der Figur 1 sind die Befestigungen 4 als Ausnehmungen bzw. Löcher ausgebildet, in welche die Stützglieder 11, 12, 13 der Figuren 4, 5, 6 eingesetzt werden können. Für jeden Finger kann ein Stützglied mit der gewünschten Krümmung, notwendiger Form und gewünschtem Querschnitt vorgesehen werden. Häufig ergibt sich der Fall, dass nur der eine oder andere Finger zu stützen ist. Die nicht zu therapierenden Finger erhalten keine Stützglieder. Sie werden im Gegensatz zu früher nicht fixiert und können daher frei bewegt werden. Für jeden Fingertyp, z.B. Zeige-, Mittel-, Ring- oder Kleinfinger ist ein Stützglied 12, 13 mit bestimmten Querschnitt, bestimmter Form und Krümmung vorgesehen. Die Stützglieder sind entweder schon fixfertig vorfabriziert oder werden mittels Epoxyharz anhand des zu therapierenden Fingers oder Daumens angefertigt. Der Finger oder der Daumen wird auf dem Stützglied, welches in die entsprechende Einrichtung 4 der Handtellerstütze 3 eingesetzt ist, in herkömmlicher Art, z.B. Binde- oder Klettenband, fixiert.

Das am Unterarm anbringbare Teil 2 der Schiene 1 und die Handtellerstütze 3 sind aus einem Stück gefertigt und können aus Kunststoffmaterial bestehen, das entweder vorgefertigt ist oder in der Praxis anhand der Masse von Unterarm und Handtellerstellung des Patienten angefertigt wird. Die Figur 1 zeigt eine Schiene 1 für einen linken Unterarm. Ohne weiteres kann eine Schiene für einen rechten Unterarm gewählt werden.

An der Aussenwand des Teils 2 der Schiene 1 ist mindestens ein Befestigungshaken 5 vorgesehen, in welchem ein Band oder ein Faden eingehakt bzw. befestigt wird, dessen anderes Ende am zu therapierenden Finger oder Daumen angebracht ist. Mit dieser Vorrichtung werden während der Mobilisationsphase die Finger und der Daumen gekrümmt. Dies erfolgt dadurch, dass der Finger oder der Daumen, entweder auf einem kurzen Stützglied 13, das nur bis zum ersten Gelenk reicht, aufliegt oder ohne Stützglied mittels des Bandes oder des Fadens in die Klauenstellung gebracht wird. Dieser Vorgang wiederholt sich so oft, wie der Therapieplan es vorschreibt. Es besteht auch die Möglichkeit, die Krümmungsänderungen der Finger und des Daumens nur mittels Stützglieder 11, 12, 13 anderer Krümmung zu bewerkstelligen, wobei die Finger und der Daumen am zugehörenden Stützglied in herkömmlicher Weise befestigt sind.

Die Figur 2 zeigt eine Schiene 1, deren Teile 2 und 3 zusammengesetzt sind. Der Vorteil dieser Ausführung ist, dass unterschiedliche Handtellerstützen 3 am Unterarmteil 2 angebracht werden können, wie z.B. eine Handtellerstütze für grosse, kleine Handflächen und rechte, linke Hände. Der genaue Winkel, der die Handtellerstütze zum Unterarm einnehmen soll, wird durch Führungsorgane 6 eingestellt. Die Führungsorgane 6 sind in der Figur 2 die Flächen des Unterarmteils 2, an welchem die Handtellerstütze zur Anlage kommt. Diese Führungsorgane 6 können einen gewünschten Neigungswinkel haben. Sie können auch als Erhöhungen 9 mit entsprechendem Neigungswinkel an der Handtellerstütze 3 vorgesehen sein. Solche Erhöhungen, die in der Figur 3 an der Handtellerstütze 3 angebracht sind, stellen keilartige Gebilde 9 mit dem gewünschten Neigungswinkel dar. Die Handtellerstütze 3 wird mit dem Unterarmteil 2 entweder fest oder lösbar verbunden. Die Klebe- oder Schraubverbindung 17 ist in der Figur 2 nur symbolisch dargestellt. Die Wahl der Verbindung hängt von den Vorstellungen des Therapeuten ab bezüglich Therapieverlauf oder Wiederverwendung der Teile 2 und 3. Wenn während des Therapieverlaufs die Winkelstellung der Hand und/oder der Finger bzw. des Daumens geändert werden muss, kann ein anderer Unterarmteil 2 oder eine andere Handtellerstütze 3 mit anders geneigten Führungsorganen 6, 9 und/oder Stützglieder mit anderen Formen, Querschnitten, Krümmungen benötigt werden. Diese Stützglieder 11, 12, 13 sind in den Figuren 4, 5, 6 gezeigt, welche mittels einer der in den Figuren 7, 8, 9 gezeigten Verbindungsvorrichtungen am oberen Rand der Handtellerstütze 3 lösbar befestigt werden können. Zu diesem Zweck werden die Ausnehmungen 4 verwendet. In üblicher Weise wird pro Stützglied ein Stift 8, 14 für ein Stützglied verwendet und in die dazugehörende Ausnehmung gesteckt. Es besteht auch die Möglichkeit, dass man für ein Stützglied zwei Stifte verwendet, welche in entsprechende Ausnehmungen geschoben werden. In diesem Falle hat es sich als zweckmässig erwiesen, dass die Stifte 8, 15 im oberen Rand der Handtellerstütze 3 angeordnet sind und in entsprechende Ausnehmungen bzw. Nuten, die in den Stützgliedern vorgesehen sind, eingreifen. Auch diese Verbindungen sind lösbar.

Die Figur 3 zeigt eine Handtellerstütze 3, an welcher ein Daumenstützteil 10 entweder an der einen oder anderen Seite angebracht wird. Hierdurch ergibt sich eine Handtellerstütze für eine rechte oder linke Hand, wobei ein und dasselbe Daumenstützteil 10 sowohlfür die eine wie auch für die andere Hand benutzt werden kann. Handtellerstütze und Daumenstützteil können aus Kunststoff oder Holz gefertigt sein. Die Figur 3 zeigt auch ein Verbindungsmittel mit zwei Stiften 8 an der Seitenfläche des Daumenstützteils 10, die in entsprechende Bohrungen 7 in der Seitenwand der Handtellerstütze 3 eingreifen. Es handelt sich um eine Verbindungsart, wie sie in der Figur 8 gezeigt ist. Das Verbindungsmittel kann auch in einer Art ausgebildet sein, wie die Figuren 7 und 9 zeigen. Handtellerstütze 3 und Daumenstützteil 10 können während des Therapieverlaufs ausgetauscht werden und nachher wieder verwendet werden.

Das Stützglied 11 für den Daumen, das in Figur 4 gezeigt ist, besteht aus Kunststoff oder Holz und ist aus einem Stück mit einer bestimmten Form, Krümmung und einem bestimmten Querschnitt gefertigt. Die verschiedenen Krümmungen, welche der Daumen während der Therapie einzunehmen hat, werden mittels Stützgliedern 11 unterschiedlicher Krümmungen bewerkstelligt, welche Stützglieder ausgetauscht werden. Das Stützglied ist an der Handtellerstütze 3 mit Hilfe eines der in den Figuren 7, 8, 9 gezeigten Verbindungseinrichtungen lösbar verbunden. Die in Figur 3 dargestellte Verbindungseinrichtung besteht aus einem Stift 8, der in das am oberen Rand der Handtellerstütze 3 angebrachte Loch 4 lösbar eingesetzt wird. Dies Bohrloch 4 ist nur symbolisch dargestellt, um die Figur 3 übersichtlich zu halten.

Das Stützglied 12 für einen Finger ist in der Figur 5 gezeigt und besteht aus Holz oder Kunststoff. Das Stützglied hat einen bestimmten Querschnitt, eine bestimmte Form, Krümmung und reicht über die gesamte Länge eines Fingers. Es ist konzipiert für Zeige-, Mittel-, Ring- sowie kleiner Finger. Die verschiedenen Krümmungen, welche die Finger während der Therapie einzunehmen haben, werden mit Hilfe der Stützglieder 12 unterschiedlicher Krümmungen erzeugt. Die Stützglieder können ausgetauscht werden. Sie sind am oberen Rand der Handtellerstütze 3 mit Hilfe einer der in den Figuren 7, 8, 9 gezeichneten Verbindungseinrichtung lösbar verbunden. Das Stützglied der Figur 5 deutet die Verbindungseinrichtung nach Figur 9 an, wonach eine Metallhülse 15 in die Bohrung 4 der Handtellerstütze 3 eingreift und mit der Schraube 16 gesichert wird.

Die Figur 6 zeigt ein Stützglied 13, das nur bis zum ersten Gelenk eines Fingers reicht und aus Holz oder Kunststoff gefertigt ist. Das Stützglied besitzt eine bestimmte Form und einen bestimmten Querschnitt, die sich nach den Fingern richten, der Zeige-, Mittel-, Ring- und kleiner Finger sein kann. Die lösbare Verbindung mit dem oberen Rand der Handtellerstütze 3 kann so ausgebildet sein, wie in den Figuren 7, 8, 9 gezeigt wurde. Die Figur 6 deutet die Verbindung nach Figur 9 an. Die Austauschbarkeit des Stützgliedes 13 ist stets gewährleistet.

In der Figur 7 ist die schwalbenschwanzförmige Verbindungseinrichtung 14 zwischen Handtellerstütze 3 und den Stützgliedern 11, 12, 13 gezeigt. Die Stützglieder werden in die Nuten eingesetzt und können zu jeder Zeit wieder aus der Nut entfernt werden, so dass ihre Austauschbarkeit gewährleistet ist. Die Nut in der Handtellerstütze 3 ist so lang wie der Schwalbenschwanz des Stützgliedes breit ist.

Die Figur 8 zeigt die Verbindungseinrichtung bestehend aus einem am Stützglied 11, 12, 13 angebrachten Stift 8, der in die im oberen Rand der Handtellerstütze 3 angeordnete Bohrung 4 eingeschoben wird. Das Stützglied kann zu jeder Zeit wieder aus der Bohrung der Handtellerstütze entfernt werden und gegebenenfalls durch ein anderes Stützglied ersetzt werden. Der Stift 8 und die Bohrung 4 sind der Einfachheit halber mit kreisförmigem Querschnitt gezeichnet. Ihre Querschnitte können auch polygon und elliptisch ausgeführt sein. Die nicht kreisförmigen Querschnitte empfehlen sich dann, wenn besonderer Wert auf eine stabile Lage der Stützglieder 11, 12, 13 relativ zur Handtellerstütze 3 gelegt wird.

Die in der Figur 9 dargestellte Verbindungseinrichtung besteht aus einer Metallhülse 15 mit einem Innengewinde, die im Stützglied 11, 12, 13 befestigt ist. Die Metallhülse 15 wird in die Bohrung 4 der Handtellerstütze 3 eingebracht und von der anderen Seite mit einer Schraube 16 in der Weise gesichert, dass die Schraube in das Innengewinde der Metallhülse 15 soweit eingeschraubt wird, dass der Schraubenkopf versenkt ist. Auch bei dieser Verbindungseinrichtung ist die Austauschbarkeit der Stützglieder 11, 12, 13 in vollem Umfang gewährleistet.

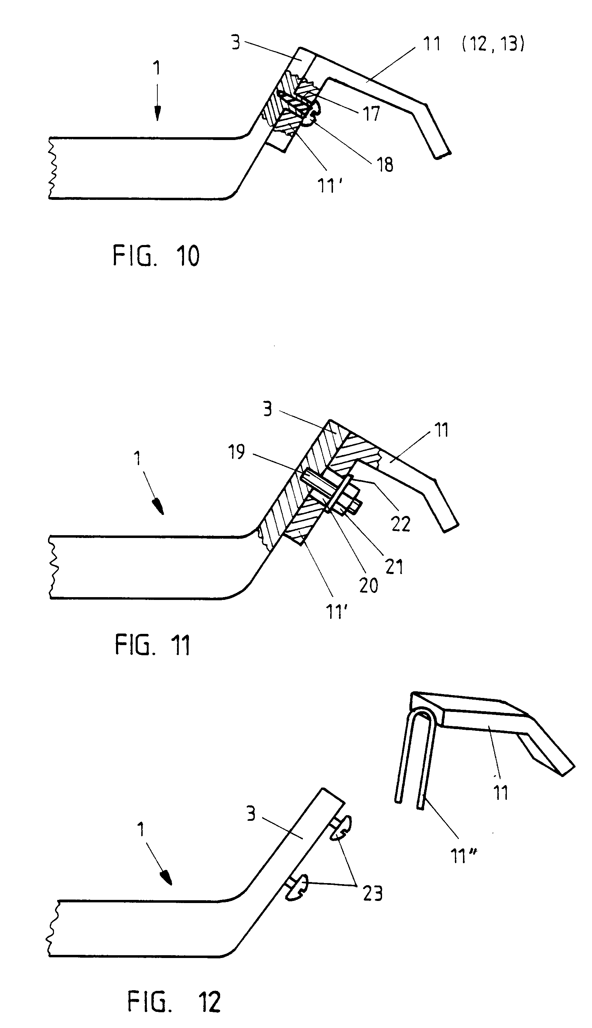

Die Figuren 10, 11 und 12 zeigen weitere Befestigungsarten für die Fingerstützglieder 11, 12 oder 13 an der Handtellerstütze 3. In Fig. 10 ist das Stützglied 11, das hier stellvertretend auch für die Stützglieder 12, 13, dargestellt ist, um einen im wesentlichen senkrecht von ihm wegragenden Arm 11' ergänzt. Der genaue Winkel dieses Arms zum Stützglied richtet sich nach der relativen Winkellage des letzteren zur Handtellerstütze 3, d.h. diese Winkellage muss gleich bleiben wie bei den Ausführungsbeispielen nach den Figuren 7-9, während der Arm 11' parallel zur Handtellerstütze 3 verläuft. Im Arm 11' befindet sich eine Bohrung 17. Durch diese wird eine Holzschraube 18 hindurchgesteckt und mittels eines Schraubenziehers in die Handtellerstütze 3 eingedreht. Der Vorteil dieser Befestigungsart liegt darin, dass man vorher die relative Lage des Stückteils 11 zur Handtellerstütze 3 genau der Hand des Patienten anpassen kann; man bringt vorher am Patienten den Stützteil in die gewünschte Lage und bohrt mit einem durch die Bohrung 17 hindurchgeführten Holzbohrer eine Vertiefung in der Handtellerstütze 3 an. Nun kann man Stütze 3 und Teil 11 vom Patienten entfernen und den Stützteil 11 mittels des Armes 11' durch Eindrehen der Schraube 18 definitiv befestigen.

Diese Art von Befestigung mittels Anschrauben des Stützteils 11 kann noch besser an den Patienten anpassbar gemacht werden, wenn man die Ausführungsform nach Fig. 11 benützt. Die Holzschraube 18 wird durch einen fest in der Handtellerstütze 3 angebrachten Gewindestift 19 ersetzt. Die Bohrung 17 wird zu einem Langloch 20 erweitert und der Arm 11' dann auf den Gewindestift 19 aufgesetzt, sodass dieser durch das Langloch 20 hindurchragt. Dank des Langloches 20 kann die definitive Lage des Stützgliedes 11 an der Handtellerstütze 3 am Patienten direkt eingestellt und hierauf durch Anziehen einer auf den Gewindestift 19 aufgesetzten Mutter 21 mit Unterlagsscheibe 22 fixiert werden. Im Gegensatz zur vorherigen Ausführungsform kann daher diese immer wieder, d.h. für verschiedene Patienten individuell einstellbar, verwendet werden.

Gleiches kann von der Ausführungsform nach Fig. 12 gesagt werden. Der Arm 11' ist jedoch durch eine U-förmige Klammer oder Spange 11'' ersetzt, die fest an derjenigen Stirnseite des Stützgliedes 11 angebracht ist, an welcher sich bei den Ausführungsformen nach den Fig. 4 - 6 der Stift 8 bzw. die Hülse 15 befindet. Die Spange 11'' kann auch durch eine längliche Platte, ähnlich dem Arm 11' ersetzt werden, welche einen U-förmigen, an ihrem frei abstehenden Ende offenen Schlitz aufweist. zwei hintereinander in die Handtellerstütze 3 eingesetzte Kopfschrauben 23, um welche sich die Spange 11'' bzw. die Platte legt, werden angezogen und halten dadurch die Spange oder Platte fest. Auch hier kann die genaue Einstellung individuell am Patienten vorgenommen und sofort fixiert werden, sodass jede nachträgliche unachtsame Verschiebung ausgeschlossen ist. Zur Verdeutlichung ist in dieser Figur das Stützglied 11 noch getrennt von der Schiene 1 und zudem in bezug auf die Handtellerstütze 3 leicht abgewinkelt dargestellt.

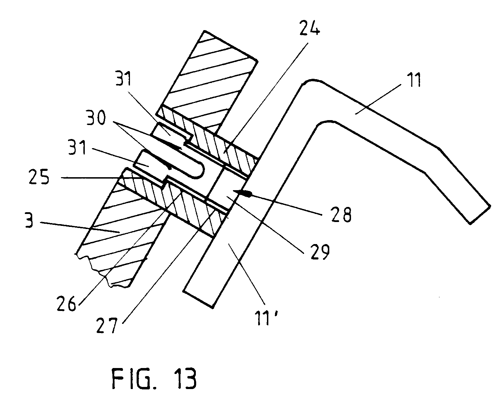

Fig. 13 schliesslich zeigt noch eine Variante der Verbindung zwischen Stützglied 11 und Handtellerstütze 3. Das Stützglied 11 weist wieder einen Arm 11' auf. In der Handtellerstütze 3 befindet sich ein annähernd würfelförmiger Körper 24 mit einer durchgehenden Bohrung, die aus drei Abschnitten 25, 26, 27 besteht. Der Abschnitt 25 ist im Querschnitt kreisrund, ebenso der Abschnitt 26, der aber einen kleineren Durchmesser hat. Der Abschnitt 27 ist quadratisch im Querschnitt. Der Arm 11' trägt einen Fortsatz 28, der einen zum Abschnitt 26 passenden Abschnitt 29, also von ebenfalls quadratischem Querschnitt, sowie zwei Zungen 30 aufweist. Diese sind an ihrer Aussenseite rund und weisen zuvorderst eine Verdickung 31 auf, die in den Abschnitt 25 passt. Da sie voneinander einen Abstand aufweisen und zudem elastisch deformierbar sind, lassen sie sich gut durch die engen Abschnitte 26 und 27 hindurchschieben. Im Abschnitt 25 weichen sie dann auseinander und verankern damit den Fortsatz 28 am Uebergang der beiden Abschnitte 25, 26. Da in dieser Stellung gleichzeitig der rechteckige Abschnitt 29 sich im ebenfalls rechteckigen Abschnitt 27 befindet, sind der Fortsatz 28 und damit das Stützglied 11 auch gegen Drehung um die Längsachse des Fortsatzes gesichert. Zum Wegnehmen des Stützgliegliedes wird auf dieses ein Zug senkrecht zur Handtellerstütze 3 ausgeübt, wodurch sich die Zungen 30 einander wieder nähern, da die Verdickung 31 an ihrem einen Ende etwas abgerundet ist, und durch die Abschnitte 26, 27 herausziehen lassen.

Infolge der vielfältigen Verbindungsmöglichkeiten der Handtellerstütze 3 am Teil 2 der Schiene 1 und mit den Stützgliedern 11, 12, 13 ist eine universelle Fixations- und Mobilisationsschiene geschaffen worden, die mit einfachen, in der Herstellung preiswerten Bauteilen die vielen während des Therapieverlaufs auftretenden Forderungen erfüllt.

## Patentansprüche

1. Fixations- und Mobilisationsschiene, welche am Unterarm befestigbar ist und eine Handtellerstütze enthält zum Fixieren eines Handtellers, wobei die Handtellerstütze von der Handwurzel bis zu den Wurzeln von Daumen und Fingern reicht und Stützmittel enthält zum Stützen des Daumens und eines jeden Fingers in einer bestimmten Stellung, **dadurch gekennzeichnet**, dass die Handtellerstütze (3) Verbindungseinrichtungen (4, 8, 14, 15, 17, 19, 23, 30, 31) enthält zum Befestigen und Austauschen von den Finger und Daumen unterstützenden Stützmittel (11, 12, 13), die für jeden Finger und den Daumen eine bestimmte gewünschte Form, Querschnitt und Krümmung aufweisen.

2. Fixations- und Mobilisationsschiene nach Anspruch 1,
**dadurch gekennzeichnet**, dass die Verbindungseinrichtungen (4, 8, 14, 15, 17, 19, 23, 30, 31) zum oberen Rand oder an anderen Bereichen der Handtellerstütze (3) angeordnet sind.

3. Fixations- und Mobilisationsschiene nach Anspruch 1, **dadurch gekennzeichnet**, dass die Handtellerstütze (3) mit der am Unterarm anliegenden Schiene (2) integriert ist und zur Schiene einen bestimmten Winkel im Bereich von 90° - 120° bildet.

4. Fixations- und Mobilisationsschiene nach Anspruch 1, **dadurch gekennzeichnet,** dass die Handtellerstütze (3) mittels Befestigungsorganen (17) an der Schiene (2) angebracht und mittels Führungsorganen (6, 9) einen bestimmten Winkel im Bereich von 90° - 120° zur Schiene bildet.

5. Fixations- und Mobilisationsschiene nach Anspruch 4, **dadurch gekennzeichnet,** dass die nur bis zu den Fingerwurzeln reichende Handtellerstütze (3) an ihren Seitenflächen Verbindungsmittel (7, 8) enthält zur Aufnahme einer Daumenstützfläche (10) für die rechte oder linke Hand.

6. Fixations- und Mobilisationsschiene nach Anspruch 1, **dadurch gekennzeichnet,** dass die am oberen Rand der Handtellerstütze (3) angebrachte Befestigungs- und Austauscheinrichtung aus mindestens einer schwalbenschwanzförmigen Ausnehmung (14) besteht, in welche das schwalbenschwanzförmige Gegenstück einschiebbar ist.

7. Fixations- und Mobilisationsschiene nach Anspruch 1, **dadurch gekennzeichnet,** dass jede Einrichtung zum Befestigen und Austauschen der Stützglieder (11, 12, 13) als eine Ausnehmung (4) kreisförmigen, polygonen oder elliptischen Querschnittes in der Handtellerstütze (3) ausgebildet ist, in welche Ausnehmung ein am Stützglied angebrachter Stift (8) entsprechenden Querschnittes eingesetzt wird.

8. Fixations- und Mobilisationsschiene nach Anspruch 6 oder 7, **dadurch gekennzeichnet,** dass bei der Einrichtung zum Befestigen und Austauschen der Stützglieder (11, 12, 13) die Ausnehmung (4, 14) am Stützglied und das in die Ausnehmung einschiebbare Gegenstück (8) an der Handtellerstütze (3) angeordnet sind.

9. Fixations- und Mobilisationsschiene nach Anspruch 1, **dadurch gekennzeichnet,** dass die Einrichtung zum Befestigen und Austauschen des Stützgliedes für einen Finger und für den Daumen aus folgenden Bauteilen besteht:
- mindestens eine Ausnehmung (4) in der Handtellerstütze (3)
- Metallhülse (15) mit Gewinde am Stützglied (11, 12, 13)
- versenkbare Schraube (16).

10. Fixations- und Mobilisationsschiene nach Anspruch 1, **dadurch gekennzeichnet,** dass die Krümmung des Stützgliedes (11, 12) der Stellung der Finger- und Daumenknochen angepasst ist.

11. Fixations- und Mobilisationsschiene nach Anspruch 1, **dadurch gekennzeichnet,** dass die Länge des Stützgliedes (11, 12) der Länge mindestens eines Finger- oder Daumenknochens entspricht.

12. Fixations- und Mobilisationsschiene nach Anspruch 1, **dadurch gekennzeichnet**, dass die Einrichtung zum Befestigen und Austauschen eines Stützgliedes (11, 12,13) einen an der Handtellerstütze 3 angeordneten Gewindestift (19) enthält, der in einem Langloch (20), das in einem Teil (11') des Stützgliedes vorgesehen ist; verschiebbar und durch ein Befestigungsmittel (21) fixierbar ist.

13. Fixations- und Mobilisationsschiene nach Anspruch 1, **dadurch gekennzeichnet**, dass die Einrichtung zum Befestigen und Austauschen aus folgenden Bauteilen besteht:
eine U-förmige Klammer (11''), welche am Stützglied (11, 12, 13) befestigt ist, wird mit ihrem an einem Ende offenen Schlitz in zwei in die Handtellerstütze (3) hintereinander eingesetzten Schrauben (23) eingeschoben, wonach die Schrauben angesogen werden.

14. Fixations- und Mobilisationsschiene nach Anspruch 1 oder Anspruch 2 , **dadurch gekennzeichnet**,
dass die Einrichtung zum Befestigen und Austauschen aus einem Schnappverschluss (24, 25, 26, 27, 28, 29, 30, 31) besteht.

## Claims

1. Fixation and mobilisation splint, to be attached to a forearm and comprising a support for fixing the palm of a hand, said support extending from the wrist of the hand to those of the thumb and the other fingers and comprising means for supporting the thumb and every other finger in a definite position, characterized in that the palm support (3) comprises connecting facilities (4, 8, 14, 15, 17, 19, 23, 30, 31) for securing and exchanging supports (11, 12, 13) for holding the thumb and the other fingers, said supports having a definite desired form, cross section and curvature adapted to every finger including the thumb.

2. Fixation and mobilisation splint according to Claim 1, characterized in that the connecting means (4, 8, 14, 15, 17, 19, 23, 30, 31) are arranged near the upper edge or on other areas of the palm support (3).

3. Fixation and mobilisation splint according to Claim 1, characterized in that the palm support (3) forms one piece with that part (2) of the splint (1) on which the forearm comes to rest and forms with that part a definite angle within the range of 90 to 120 degrees.

4. Fixation and mobilisation splint according to Claim 1, characterized in that the palm support (3) is attached to the splint (2) by means of fixation organs (17) and forms an angle with it in the range of 90 to 120 degrees with the aid of guiding organs (6, 9).

5. Fixation and mobilisation splint according to Claim 4, characterized in that the palm support (3) extending to the finger roots comprises connecting means (7, 8) at their lateral sides for receiving a thumb support (10) for the right or left hand.

6. Fixation and mobilisation splint according to Claim 1, characterized in that the fixation and exchange facility disposed at the upper edge of the palm support (3) consists of at least one dove-tailed cavity (14) into which fills the dove-tailed counterpart of a finger support.

7. Fixation and mobilisation splint according to Claim 1, characterized in that each facility for fixing and exchanging the finger supports (11, 12, 13) is formed in the palm support (3) as a cavity (4) of circular, polygonal or elliptic cross section into which is inserted a pin (8) attached to the finger support and having a corresponding cross section.

8. Fixation and mobilisation splint according to Claim 6 or 7, characterized in that on the facility for fixing and exchanging the finger supports (11, 12, 13) the cavity (4, 14) is provided at the respective support whereas the counterpart (8) to be inserted into the cavity is attached to the palm support (3).

9. Fixation and mobilisation splint according to Claim 1, characterized in that the facility for fixing and exchanging the finger support comprises the following items:
- at least one cavity (4) in the palm support (3)
- a metal sleeve (15) having a thread and being secured to the finger support (11, 12, 13)
- a countersunk screw (16).

10. Fixation and mobilisation splint according to Claim 1, characterized in that the curvature of the finger support (11, 12) is adapted to the position of the bones of the thumb or of the other fingers.

11. Fixation and mobilisation splint according to Claim 1, characterized in that the length of the finger support (11, 12) corresponds to the length of at least one bone of the thumb or of any other finger.

12. Fixation and mobilisation splint according to Claim 1, characterized in that the facility for fixing and exchanging a finger support (11, 12, 13) comprises a threaded pin (19) attached to the palm support (3) which is displaceable relative to an oblong hole (20) arranged in a part (11') of the finger support; enabling the latter to be fixed on said pin by means of a fixing device (21).

13. Fixation and mobilisation splint according to Claim 1, characterized in that the facility for fixing and exchanging a finger support consists of the following items:
- a U-shaped clasp (11'') secured to the finger support (11, 12, 13), and forming a slot between its legs;
- two head screws (23) arranged in line in the palm support (3), the clasp (11'') surrounding said screws and becoming fixed by their heads after having screwed them down.

14. Fixation and mobilisation splint according to Claim 1 or 2, characterized in that the facility for fixing and exchanging comprises a spring catch (24, 25, 26, 27, 28, 29, 30, 31).

## Revendications

1. Eclisse de fixation et de mobilisation, laquelle peut se fixer sur l'avant-bras et contient un appui de paume pour fixer la paume d'une main, l'appui de paume allant de la base de la main jusqu'aux bases du pouce et des doigts et contient un moyen de soutien pour soutenir le pouce et chaque doigt dans une position déterminée, caractérisée en ce que l'appui de paume (3) contient des dispositifs de liaison (4, 8, 14, 15, 17, 19, 23, 30, 31) pour fixer et échanger les moyens de soutien (11, 12, 13) des doigts et du pouce, qui présentent pour chaque doigt et le pouce certaines forme, section et courbure désirées.

2. Eclisse de fixation et de mobilisation selon la revendication 1, caractérisée en ce que les dispositifs de liaison (4, 8, 14, 15, 17, 19, 23, 30, 31) sont disposés sur le bord supérieur ou dans d'autres zones de l'appui de paume (3).

3. Eclisse de fixation et de mobilisation selon la revendication 1, caractérisée en ce que l'appui de paume (3) est intégré à l'éclisse (2) placée sur l'avant-bras et forme par rapport à l'éclisse un certain angle dans le domaine de 90°-120°.

4. Eclisse de fixation et de mobilisation selon la revendication 1, caractérisée en ce que l'appui de paume (3) est apposé sur l'éclisse (2) au moyen d'organes de fixation (17) et forment par rapport à l'éclisse, au moyen d'organes de guidage (6, 9), un certain angle dans le domaine de 90°-120°.

5. Eclisse de fixation et de mobilisation selon la revendication 4, caractérisée en ce que l'appui de paume (3) n'allant que jusqu'à la base des doigts, contient sur ses surfaces latérales des moyens de liaison (7, 8) pour recevoir une surface d'appui de pouce (10) pour la main droite ou gauche.

6. Eclisse de fixation et de mobilisation selon la revendication 1, caractérisée en ce que le dispositif de fixation et d'échange placé sur le bord supérieur de l'appui de paume (3) est constitué d'au moins un creux en forme de queue d'aronde (14), dans laquelle la partie correspondante en queue d'aronde peut s'engager.

7. Eclisse de fixation et de mobilisation selon la revendication 1, caractérisée en ce que chaque dispositif de fixation et d'échange des organes de soutien (11, 12, 13) est constitué en tant que creux (4) de section circulaire, polygonale ou elliptique dans l'appui de paume (3), dans lequel creux un ergot (8) de section correspondante placé sur l'organe de soutien est enfoncé.

8. Eclisse de fixation et de mobilisation selon la revendication 6 ou 7, caractérisée en ce que, dans le dispositif de fixation et d'échange des organes de soutien (11, 12, 13), le creux (4, 14) sur l'organe de soutien et la partie correspondante (8) s'enfonçant dans le creux sont placés sur l'appui de paume (3).

9. Eclisse de fixation et de mobilisation selon la revendication 1, caractérisée en ce que le dispositif de fixation et d'échange de l'organe de soutien pour un doigt et pour le pouce est constitué des éléments suivants:
- au moins un creux (4) dans l'appui de paume (3),
- un manchon métallique (15) avec pas de vis sur l'organe de soutien (11, 12, 13),
- une vis à tête conique (16).

10. Eclisse de fixation et de mobilisation selon la revendication 1, caractérisée en ce que la courbure de l'organe de soutien (11, 12) est adaptée à la position de l'os du doigt et du pouce.

11. Eclisse de fixation et de mobilisation selon la revendication 1, caractérisée en ce que la longueur de l'organe de soutien (11, 12) correspond à la longueur d'au moins un os de doigt ou de pouce.

12. Eclisse de fixation et de mobilisation selon la revendication 1, caractérisée en ce que le dispositif de fixation et d'échange d'un organe de soutien (11, 12, 13) contient un ergot fileté (19) placé sur l'appui de paume (3) et qui s'engage dans un trou oblong (20) qui est prévu dans une partie (11') de l'organe de soutien, et peut y être fixé par un moyen de fixation (21).

13. Eclisse de fixation et de mobilisation selon la revendication 1, caractérisée en ce que le dispositif de fixation et de soutien est constitué des éléments suivants:
une agrafe en forme de U (11''), qui est fixée sur l'organe de soutien (11, 12, 13), est enfoncée avec sa fente ouverte à une extrémité dans deux vis (23) insérées successivement dans l'appui de paume (3), après quoi les vis sont serrées.

14. Eclisse de fixation et de mobilisation selon la revendication 1 ou la revendication 2, caractérisée en ce que le dispositif de fixation et d'échange est constitué d'un bouchon à encliquetage (24, 25, 26, 27, 28, 29, 30, 31).
